# EUROPEAN PATENT APPLICATION

(11) **EP 3 566 831 A1**
(43) Date of publication of application: **13.11.2019**
(21) Application number: 17890146.8
(22) Date of filing: 01.12.2017
(51) Int. Cl.: B26B 19/48

(54) **INTELLIGENT HOOD AND CONTROL METHOD THEREFOR, AND TERMINAL**

(30) Priority: 03.01.2017 CN 201710001153
(71) Applicant: BOE Technology Group Co., Ltd., Beijing 100015 (CN); Chengdu BOE Optoelectronics Technology Co., Ltd., Chengdu, Sichuan 611731 (CN)
(72) Inventor: LIU, Tingliang, Beijing 100176 (CN); ZANG, Pengcheng, Beijing 100176 (CN); XU, Yuanjie, Beijing 100176 (CN); HUANG, Weiyun, Beijing 100176 (CN); LI, Ting, Beijing 100176 (CN); GUO, Wei, Beijing 100176 (CN)
(74) Representative: Klunker IP Patentanwälte PartG mbB
(86) International application number: PCT/CN2017/114277
(87) International publication number: WO 2018/126827

(57) **Abstract**

An intelligent hood (200), comprising: a plurality of sensors (210), a hairstyle processing assembly (220), and a control unit (230); wherein a plurality of sensors (210) are configured to acquire sensing data related to a user; and the control unit (230) is configured to control the hairstyle processing assembly (220) to execute a hairstyle processing action based on a control instruction, wherein the control instruction is generated based on the sensing data, and the intelligent hood (200) can cut a hairstyle closer to the desired hair style of a customer.

## Description

### TECHNICAL FIELD

Embodiments of the present disclosure relate to an intelligent head cover and a control method thereof, and a terminal.

### BACKGROUND

In a current social life, people need to look for a barber to cut hair for different hairstyles, and spend a lot of time on washing and cutting hair. Sometimes, because a hairstyle designed by a hairdresser is far from a desired hairstyle, it is difficult to meet a customer's expectation.

### SUMMARY

At least an embodiment of the disclosure provides an intelligent head cover, comprising: a sensor, configured to acquire sensory data related to a user; a hairstyle processing component; and a controller, configured to control the hairstyle processing component to perform a hairstyle processing action based on a control instruction, wherein: the control instruction is generated based on the sensory data.

For example, the intelligent head cover further comprises a communication unit, wherein: the communication unit is configured to send the sensory data and receive the control instruction, and the control instruction includes a hair cutting instruction for controlling turning on or off of the hairstyle processing component.

For example, the intelligent head cover further comprises an instruction generator, wherein: the instruction generator is configured to generate the control instruction based on the sensory data, the control instruction includes a hair cutting instruction for controlling turning on or off of the hairstyle processing component.

For example, the intelligent head cover further comprises a communication unit, an instruction generator and a selector, wherein: the communication unit is configured to establish connection with a terminal. The selector is configured to: instruct the communication unit to receive the control instruction from the terminal, when the communication unit successfully establishes connection with the terminal; instruct the instruction generator to generate the control instruction, when the communication unit fails to establish connection with the terminal. When the communication unit successfully establishes connection with the terminal, the communication unit is further configured to send the sensory data to the terminal and receive the control instruction from the terminal, the control instruction is generated based on the sensory data by the terminal; or when the communication unit fails to establish connection with the terminal, the instruction generator is configured to generate the control instruction based on the sensory data. The control instruction includes a hair cutting instruction for controlling turning on or off of the hairstyle processing component.

For example, the plurality of sensors includes: a plurality of cameras; the sensory data includes: images of the user's head taken by the plurality of cameras; and the images of the user's head include a combination of one or more images of a head form, a facial form and hair distribution of the user.

For example, at least one of the plurality of cameras is located in the front of an interior of the head cover, the front is a position close to a face when the intelligent head cover is worn; and at least one of the plurality of cameras is located in the back or on the top of the interior of the intelligent head cover, the back is a position away from the face when the intelligent head cover is worn, and the top is a position close to a head vertex when the intelligent head cover is worn.

For example, the plurality of sensors includes a temperature sensor; and the sensory data further includes a temperature inside the intelligent head cover sensed by the temperature sensor.

For example, the hairstyle processing component includes a hair cutting component; the hair cutting component includes: razors or combs distributed on an inner surface of the intelligent head cover; the razors are configured to: cut the user's hair based on the hair cutting instruction; and the combs are configured to: comb the user's hair based on the hair cutting instruction.

For example, the hair cutting instruction further includes position information of the razors and the combs.

For example, the hairstyle processing component includes a dyeing component; the dyeing component further includes: pipes and a storing unit distributed in a mesh structure on an inner surface of the intelligent head cover; the storing unit is configured to store a dyeing material.

For example, the control instruction further includes a dyeing instruction; the controller is configured to control opening or closure of the storing unit based on the dyeing instruction; and the storing unit is further configured to provide the dyeing material to the pipes when opened.

For example, a heating unit is configured to generate heat and provide temperature preservation inside the intelligent head cover under control of a heating instruction.

For example, the intelligent head cover further comprises: a massage unit located inside the intelligent head cover, and configured to perform massage on the user's head under control of a massage instruction.

For example, the intelligent head cover further comprises: a hair quality detecting unit, configured to detect the user's hair quality.

For example, the intelligent head cover further comprises an advising unit and a display unit, wherein: the advising unit is configured to generate advice information based on the sensory data, the advice information including at least one of a hairstyle advice, a dyeing advice, a massage advice, and a health condition advice; or, the advising unit is configured to receive the advice information; and the display unit is located in the front of the intelligent head cover, and is configured to display the advice information to the user.

Embodiments of the disclosure provide a control method of the intelligent head cover described above, comprising: acquiring sensory data related to a user; and controlling a hairstyle processing component to perform a hairstyle processing action based on a control instruction.

Embodiments of the disclosure provide a terminal, comprising: a communication unit, configured to receive sensory data related to a user; and a generating unit, configured to generate a control instruction according to the sensory data, the control instruction being used for controlling a hairstyle processing component in an intelligent head cover to perform a hairstyle processing action; wherein: the communication unit is further configured to send the control instruction to the intelligent head cover.

For example, the terminal further comprises a hairstyle designing unit, configured to provide a hairstyle advice based on the sensory data related to the user, wherein the hairstyle designing unit includes: a processing module, configured to process the sensory data to generate a head form parameter, a facial form parameter or a hair parameter of the user; and a matching module, configured to match the head form parameter, the facial form parameter or the hair parameter of the user with data of a hairstyle database, to obtain a hairstyle advice including at least one recommended hairstyle; and a providing module, configured to provide the user with the hairstyle advice.

For example, the control instruction includes a massage instruction for controlling a massage unit of the intelligent head cover and a heating instruction for controlling a heating unit of the intelligent head cover; and the generating unit is further configured to generate the hair cutting instruction, the massage instruction and the heating instruction.

For example, the terminal further comprises an analyzing unit, configured to: acquire a hair quality parameter of the user according to the sensory data, the hair quality parameter including a hair color and a hair elasticity; and acquire the user's health condition according to the hair quality parameter, and provide a health condition advice.

Embodiments of the disclosure provide a processing method for the terminal described above, which may comprise: receiving sensory data related to a user from an intelligent head cover; providing a hairstyle advice according to the sensory data; generating a control instruction according to the sensory data, wherein the control instruction includes a hair cutting instruction for controlling turning on or off of a hair cutting component in the intelligent head cover; and sending the control instruction to the intelligent head cover.

For example, the processing method further comprises: acquiring the user's hair quality parameter according to the sensory data, the hair quality parameter including a hair color and a hair elasticity; and acquiring the user's health condition according to the hair quality parameter, and providing an advice for improvement.

### BRIEF DESCRIPTION OF THE DRAWINGS

In order to illustrate the technical solutions in the embodiments of the present disclosure more clearly, the drawings used in the description of the embodiments will be briefly described in the following; it is obvious that the drawings described below are only related to some embodiments of the present disclosure, and not intended to be limitative to the disclosure.
FIG. 1 is a schematic diagram of an application scenario of a technical solution provided by an embodiment of the present disclosure;
FIG. 2 is a block diagram of an intelligent head cover provided by an embodiment of the present disclosure;
FIG. 3 is a diagram of a physical example of an intelligent head cover provided by an embodiment of the present disclosure;
FIG. 4A is a flow chart of a control method of an intelligent head cover provided by an embodiment of the present disclosure;
FIG. 4B is a flow chart of a method for controlling operation of a hair cutting component provided by an embodiment of the present disclosure;
FIG. 5 is a block diagram of a terminal provided by an embodiment of the present disclosure;
FIG. 6 is a block diagram of a hairstyle designing unit located at a terminal provided by an embodiment of the present disclosure;
FIG. 7A to FIG. 7C are schematic diagrams of 3 types of interfaces located on a terminal provided by an embodiment of the present disclosure; and
FIG. 8 is a flow chart of a processing method on a terminal provided by an embodiment of the present disclosure.

### DETAILED DESCRIPTION

Hereinafter, the technical solutions of the embodiments of the present disclosure will be described in a clearly and fully understandable way in conjunction with the drawings related to the embodiments of the present disclosure; with reference to non-restrictive exemplary embodiments shown in the drawings and described in detail in the following description, exemplary embodiments of the present disclosure and their various features and favorable details are illustrated more comprehensively. It should be noted that, the features shown in the drawings are not necessarily drawn according to scale. Known materials, components and process technologies are not described in the present disclosure so as not to obscure the exemplary embodiments of the present disclosure. Examples given are merely intended to facilitate understanding of implementation of exemplary embodiments of the present disclosure, and further enable those skilled in the art to implement the exemplary embodiments. Therefore, the examples should not be construed as limiting the scope of the exemplary embodiments of the present disclosure.

Unless otherwise defined, technical terms or scientific terms used in the present disclosure should be of general meaning as understood by those ordinarily skilled in the art. "First", "second" and similar words used in the present disclosure do not represent any sequence, quantity or importance and merely intend to differentiate different composite parts. In addition, in respective embodiments of the present disclosure, same or similar reference signs denote same or similar parts.

Embodiments of the present disclosure may achieve a relaxed and enjoyable hair cutting experience for a user, to provide a hairstyle that is closer to a desired hairstyle, and even further implement functions such as perming and dyeing. At a same time, the embodiments of the present disclosure may implement a massage function on a user's head. In addition, hair is an indicator for a state of a human life and physical body, and hair is closely related to diseases, and increasingly receives attention from medical workers. Through the embodiments of the present disclosure, a user can easily and clearly obtain a hair quality condition of his/her own and detect a physical health condition at any time, and the user's hair quality condition can be fed back to the user through an external mobile terminal device.

Hereinafter, an intelligent head cover, a control method thereof and a terminal as well as a processing method thereof provided by the embodiments of the present disclosure are described in detail in conjunction with the drawings.

The embodiments of the present disclosure provide a terminal installed with an application (APP) to implement functions such as hairstyle design and hair quality detection. For example, in the embodiments of the present disclosure, hairstyle design is implemented with the terminal, and the designed hairstyle is sent to a corresponding intelligent head cover worn by a user, so as to implement rapidity and differentiation of hair cutting, which may be more convenient to the user having a hair cutting demand. The embodiments of the present disclosure may implement connection between a terminal device and an intelligent head cover, and easily implement remote management and interoperability of the terminal device and the intelligent head cover. The embodiments of the present disclosure may further implement detection of the hair quality of the user wearing the intelligent head cover, which facilitates the user to learn his/her own health condition in time.

For example, the terminal may be a control terminal of the intelligent head cover, and the user may control various working units deployed inside the intelligent head cover with the terminal through a network such as 3G/4G and WIFI. The embodiments of the present disclosure perform differentiated hairstyle design based on sensory data received from the intelligent head cover through an application (app) of the terminal. In order to design an appropriate hairstyle, the intelligent head cover may collect image data for information such as a facial form and a hairstyle of the user wearing the intelligent head cover, and the application of the terminal is further used for implementing interactive communication between the user and the hardware.

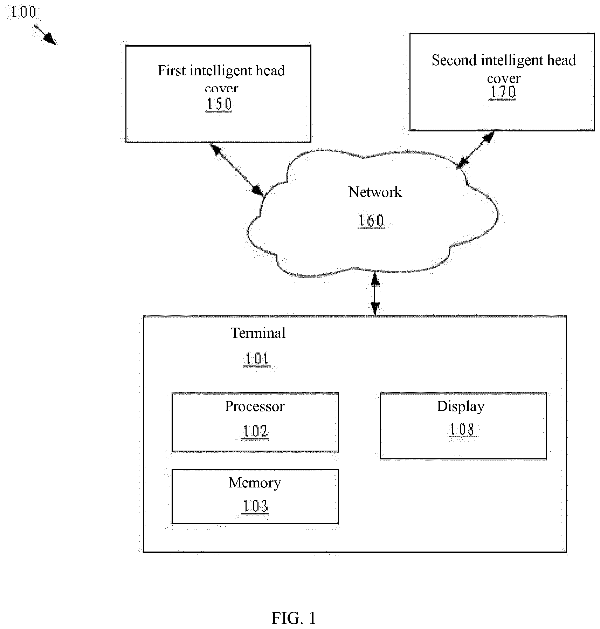

As shown in FIG. 1, a system 100 for implementing functions provided by an embodiment of the present disclosure comprises: a terminal 101, a first intelligent head cover 150 and a second intelligent head cover 170; wherein the terminal 101 may complete control of the first intelligent head cover 150 and the second intelligent head cover 170 with an intelligent server (not shown in FIG. 1) (for example, sending an instruction to control turning ON or OFF of a hair cutting component to the intelligent head cover). FIG. 1 only shows the first intelligent head cover 150 and the second intelligent head cover 170, and in an actual scenario, the terminal 101 may be connected with more intelligent head covers to control operations of the more intelligent head covers. For example, if a network 160 is a Bluetooth wireless network, the terminal 101 may be used as a primary device, and the primary device may control operations of a plurality of intelligent head covers. For another example, if the network 160 is a 3G access network, the terminal 101 may mutually communicate with more intelligent head covers. The intelligent server may also be respectively connected with the respective intelligent head covers and the terminal 101 via the network 160.

The first intelligent head cover 150 may be configured to: perform a hair cutting operation on a user, in response to a control instruction generated by the terminal 101, or in response to receiving a recommended hairstyle from the terminal 101. For example, the first intelligent head cover 150 may complete an operation specified by the control instruction, in response to receiving the control instruction (e.g., controlling the hair cutting component to be turned on, or controlling a heating unit to provide heat). For another example, the first intelligent head cover 150 may perform a corresponding hair cutting operation according to the recommended hairstyle, in response to the received recommended hairstyle. Correspondingly, the second intelligent head cover 170 and other intelligent head covers not shown in FIG. 1 may also complete same or similar functions as the first intelligent head cover 150, which will not be repeated in the present disclosure.

In some embodiments, a first intelligent head cover 150 may further generate a control instruction by itself. In this case, the first intelligent head cover does not rely on a terminal 101 to provide a needed control instruction.

In some embodiments, a terminal 101, a first intelligent head cover 150 and a second intelligent head cover 170 implement interconnection through a network 160. For example, the network 160 includes, but is not limited to, a mobile communication access network (e.g., 4G, 3G), a wide area network, or a local area network (e.g., WIFI or Bluetooth), and the like. For another example, the network 160 includes the Internet.

For example, the terminal 101 may be a computing device that includes a processor and a memory. For example, the terminal 101 may be a smartphone, a computer, and other terminals (e.g., a mobile terminal) including a processor and a memory. In some embodiments, the terminal 101 may comprise a processor 102, a memory 103, a display 108, a browser (not shown), an input device (e.g., a microphone) and other output devices (e.g., a speaker), and other components.

The processor 102 may process a data signal, and may include various computation structures, for example, a complex instruction set computer (CISC) structure, a reduced instruction set computer (RISC) structure, or a structure that implements a combination of various instruction sets. In some embodiments, the processor 102 may also be a microprocessor.

The memory 103 may store instructions and/or data executed by the processor 102. These instructions and/or data may include codes, for implementing some or all of the functions of one or more modules as described in the embodiments of the present disclosure. For example, the memory 103 includes a dynamic random access memory (DRAM), static random access memory (SRAM), flash memory, optical memory, or other memory known to those skilled in the art.

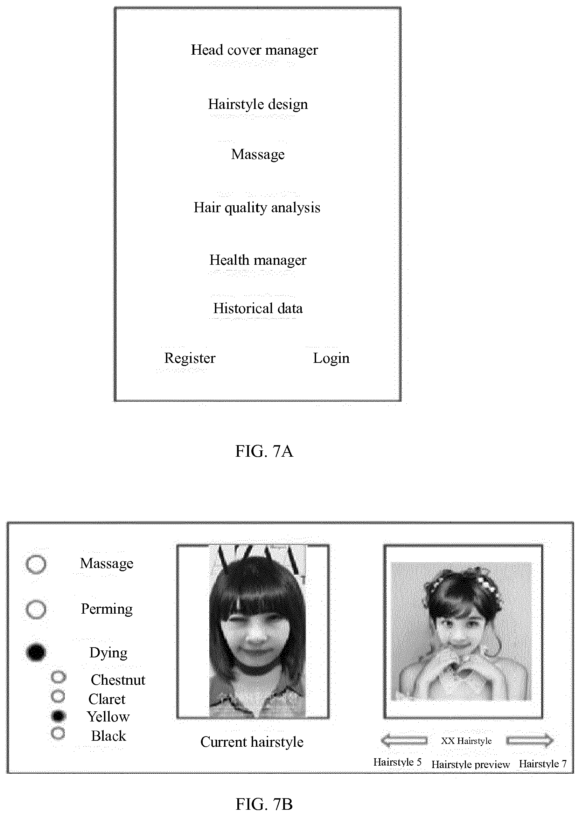

The display 108 may be used for displaying data. For example, the display 108 may be an LCD display, an LED display, or other displays. The display 108 may include a display screen with a touch function. For example, the display 108 may display a hairstyle recommended to the user, and may also receive interactive information input by the user. In some examples, the display 108 may further display information as shown in FIG. 7A to FIG. 7C to the user.

In some embodiments, an intelligent head cover (e.g., a first intelligent head cover 150 or a second intelligent head cover 170) may comprise components similar to a terminal 101, for example, a display, a processor and a memory (not shown); and related functions of the processor and the memory of the intelligent head cover are similar to those of a processor 102 and a memory 103 of the terminal 101 respectively.

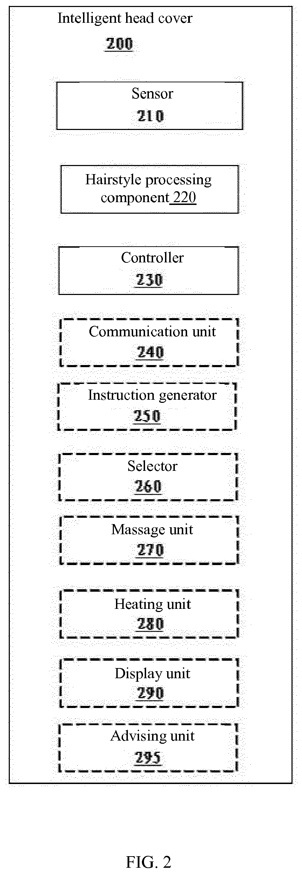

As shown in FIG. 2, an embodiment of the present disclosure provides an intelligent head cover 200. The intelligent head cover 200 may comprise: a sensor 210, a hairstyle processing component 220, and a controller 230. The sensor 210 is configured to acquire sensory data related to a user. The controller 230 is configured to control the hairstyle processing component 220 to perform a hairstyle processing action based on a control instruction, wherein the control instruction is generated based on the sensory data.

In some embodiments, the intelligent head cover 200 may further comprise a communication unit 240, wherein the communication unit 240 is configured to send sensory data and receive a control instruction, wherein the control instruction is generated based on the sensory data, and the control instruction includes: a hair cutting instruction for controlling turning ON or OFF of the hairstyle processing component 220.

For example, the communication unit 240 may be a wireless transceiver unit. The wireless transceiver unit may also be used for sending the sensory data collected by the sensor, such as a camera, to a terminal, and may also receive the generated control instruction, and the like, from the terminal 101. The communication unit 240 implements communication with the terminal 101 by using a corresponding communication protocol. For example, if a network shown in FIG. 1 is a Bluetooth network, the communication unit 240 communicates with the terminal 101 shown in FIG. 1 by using a Bluetooth protocol. For example, the control instruction received by the communication unit 240 may at least include a control instruction such as a dyeing instruction.

In some embodiments, the intelligent head cover 200 may further comprise an instruction generator 250, wherein the instruction generator 250 is configured to generate a control instruction based on sensory data, and the control instruction includes a hair cutting instruction for controlling turning ON or OFF of the hairstyle processing component 220.

In some embodiments, the intelligent head cover 200 may further comprise simultaneously: the communication unit 240, the instruction generator 250 and a selector 260. In this case, the communication unit 240 is configured to establish connection with a terminal. The selector 260 is then configured to: when the communication unit 240 successfully establishes connection with the terminal (for example, a terminal 101 shown in the diagram), instruct the communication unit 240 to receive a control instruction from the terminal; when the communication unit 240 fails to establish connection with the terminal, instruct the instruction generator 250 to generate a control instruction; wherein, when the communication unit 240 successfully establishes connection with the terminal, the communication unit 240 is further configured to send sensory data to the terminal and receive the control instruction from the terminal, and the control instruction is generated based on the sensory data; or, when the communication unit 240 fails to establish connection with the terminal, the instruction generator 250 is configured to generate the control instruction based on the sensory data.

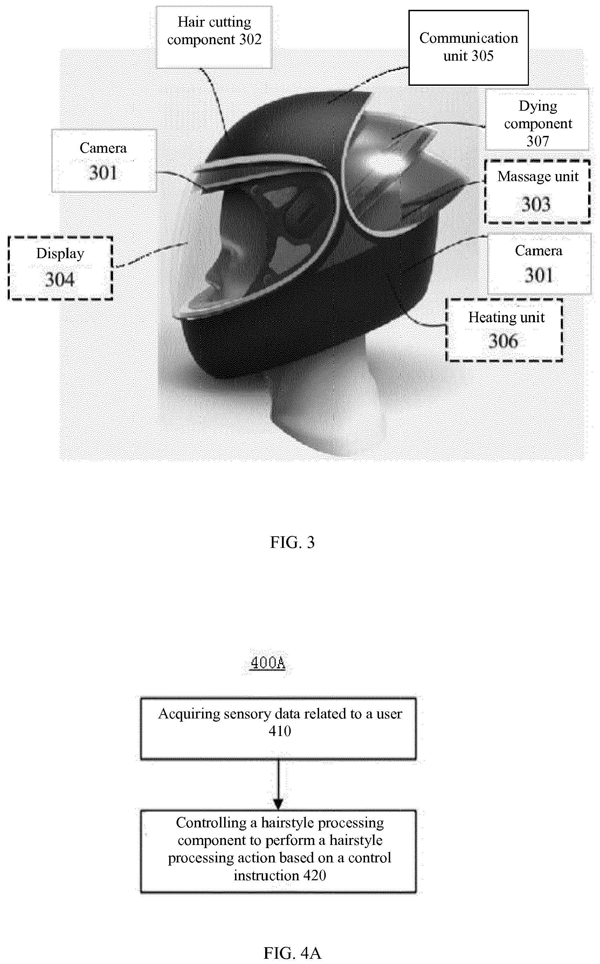

In some embodiments, a plurality of sensors 210 in an intelligent head cover 200 may further include: a plurality of cameras (for which FIG. 3 may be referred to). In this case, sensory data includes: an image of a user's head taken by the plurality of cameras; and the image of the user's head may further include a combination of one or more images of a head form, a facial form and hair distribution of the user.

For example, at least one of the plurality of cameras is located in the front of an interior of the head cover, wherein the front is a position close to the face when the intelligent head cover is worn; at least one of the plurality of cameras is located in the back or on the top of the interior of the intelligent head cover, wherein the back is a position away from the face when the intelligent head cover is worn, and the top is a position close to the head vertex when the intelligent head cover is worn. A camera located in the front of the interior of the intelligent head cover 200 is used for acquiring images of the user's face and images of the front half of the head, and the images may be used for determining the head form, a hair density condition, and other parameters subsequently. Another camera located in the back or on the top of the interior of the intelligent head cover is used for acquiring images of a rear half of the user's head; and the images of the rear half may also be used for acquiring the head form, a hair length, and other parameters subsequently. In addition, a camera located in front of the head on its own may also be used for capturing an image of the wearer's facial form. In addition, three cameras may be set respectively, wherein a first camera is used for acquiring images of the facial form, a second camera is used for acquiring the images of the front half of the head form, a hair density, and the like, and a third camera is used for acquiring images of the rear half of the head form, as well as a current hair length and color, and the like. In embodiments of the present disclosure, the images taken by the cameras may be regarded as part of the sensory data collected by the intelligent head cover 200.

For example, a camera may be a digital camera, or may be an analog camera, wherein the digital camera may convert an analog video signal generated by a video capturing device into a digital signal, and further store the same in a computer. A video signal captured by the analog camera may be converted into a digital form and compressed by a specific video capture card before it is used by a computer. The digital camera may capture an image directly, and then send the image to the computer through a serial port, a parallel port or a USB interface.

For example, the camera generally has basic functions such as video camera/broadcast and static-image capture. After an image is captured by a lens, the image is processed by a photosensitive circuit and a control unit in the camera, converted into the digital signal that may be recognized by the computer, and then input into the computer through the parallel port or USB connection, to be reconstructed by software.

In some embodiments, the plurality of sensors comprised by the intelligent head cover 200 includes a temperature sensor; and in this case, the sensory data further includes temperature data inside the intelligent head cover 200 sensed by the temperature sensor.

In some embodiments, the hairstyle processing component 220 may include a hair cutting component (for which FIG. 3 may be referred to); and the hair cutting component may further include: razors or combs (not shown) distributed on an inner surface of the intelligent head cover 200. In this case, the razors are configured to cut a user's hair based on a hair cutting instruction; and the combs are configured to comb the user's hair based on the hair cutting instruction. For example, the hair cutting instruction further includes position information of the razors and the combs. For example, the razors may be evenly distributed on an inner surface of the intelligent head cover 200 that is corresponding to the hair distribution, and the number of combs may be less than the number of razors. For another example, a comb may be rotated by a certain angle along an axis, or may be shifted by a certain distance on the inner surface of the head cover, so that an area covered by the comb when combing the hair may be enlarged. For another example, the combs and the razors may be controlled not to operate simultaneously by a control instruction, the combs are in an OFF state when the razors are cutting hair, and the combs may be set to comb hair when the razors are not operating. For example, corresponding identifiers and addresses may be assigned to the razors or the combs by a controller 230 in advance. After receiving the control instruction from a terminal 101, the controller 230 may identify corresponding combs or razors by addressing, then send a control instruction to the corresponding combs or razors through a data line, to control turning on or off of the razors or the combs, and to further control a turn-on duration of each respective razor.

In some embodiments, positions of the razors and the combs, as well as the turn-on durations of the razors and the combs, and cutting degrees (amounts of movement of the razors and the combs) may be obtained according to the sensory data and a hair cutting degree value pre-set by a user).

In some embodiments, a related hair cutting instruction may be generated according to a hairstyle advice. For example, after a hairstyle is selected in a first stage, a model needs to be created; the model is completed by splicing patterns of each unit area (1 cm² to 5 cm²), and different unit areas or starting point positions are selected by an approach similar to mathematical iteration or by a quadratic model, so as to further revise and refine the created initial model, and the model is set to be f(x)=f(x1, x2, x3... xn). After the model is created, by calculation and analysis, a cutting amount required for each corresponding unit is obtained. By taking a position of f(x1) as an example, the total number of hairs is m, a length distribution is g(l1, l2, l3...lm), and an objective desired to achieve is that the length is changed to g'(L1, L2, L3... Lm), such that when the power supply is turned on, the razors and combs in the region will complete this cutting task. Since the cutting task is from g(l1,l2,l3...lm) to g'(L1, L2, L3...Lm), a cutting amount of each hair is not equal, and a subsequent measurement analysis is needed after completing cutting each hair, and cutting of the current hair may stop after it is confirmed that the hair's cutting amount is completed.

In some embodiments, the hairstyle processing component 220 includes a dyeing component (as shown in FIG. 3). The dyeing component may further include: pipes and storing units (not shown) distributed in a mesh structure on an inner surface of the head cover; wherein each storing unit is configured to store a dyeing material. For example, a storing unit may include a storage tank or a storage capsule, and the like. For example, the dyeing component includes a brush, and dye in the pipes may be provided to the brush to perform dyeing.

Corresponding to the example of the above-described dyeing component, a control instruction may further include a dyeing instruction. In this case, the controller 230 is configured to control turning ON or OFF of the storing units included in the dyeing component based on the dyeing instruction; and the storing units are further configured to provide the dyeing material to the pipes when being opened.

In some embodiments, the intelligent head cover 200 may further comprise: a heating unit 280. The heating unit 280 is configured to generate and preserve heat inside the head cover under control of a heating instruction.

For example, the heating unit 280 is an electric heating device. The heating unit 280 may be used in combination with a heat dissipation device, so as to implement rapid and uniform dissipation of heat generated within the head cover. In addition, in order to ensure that the heating unit 280 works more safely, a safety control device is further provided for the heating unit 280, and the safety control device may sense a temperature of the heating unit 280. When the temperature of the heating unit 280 sensed by the safety control device is significantly higher than a temperature that may be tolerated by a person, the power supply will be turned off to stop generating heat. For example, the safety control device may use a positive temperature coefficient thermistor.

In some embodiments, the intelligent head cover 200 may further comprise: a massage unit 270 (for which FIG. 3 may also be referred to) located inside the head cover, and the massage unit 270 is configured to perform massage on a user's head under control of a massage instruction. For example, the massage unit 270 drives a flexible pad to vibrate by a motor, so as to perform massage.

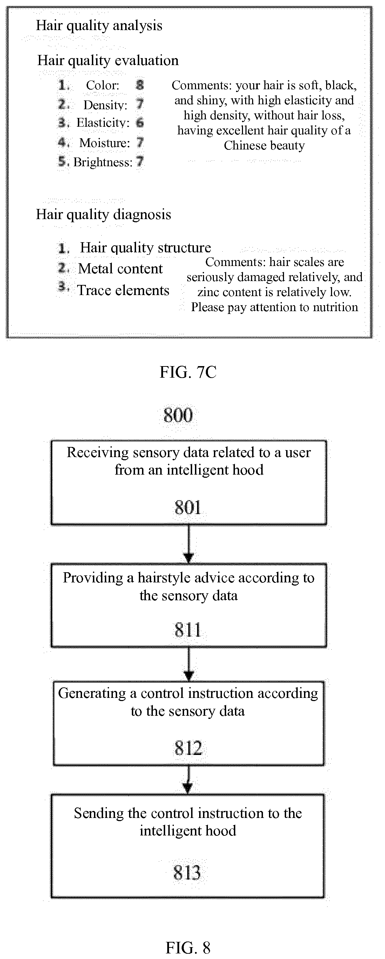

In addition, the intelligent head cover 200 may further comprise a hair quality detecting unit configured to detect the user's hair quality, and the hair quality detecting unit may be further configured to execute other processing operations related to the hair quality as shown in FIG. 7C.

For example, the massage unit 270 is an electronic massage device, and a massage time and a massage force of the massage unit 270 may be adjusted. For example, the terminal shown in FIG. 1 may generate a control instruction, or the instruction generator 250 of the intelligent head cover may provide the control instruction, to control the turning ON or OFF of the massage unit 270 and to control a massage force of the massage unit 270 according to the control instruction. In addition, the massage force may also be manually set or adjusted through an interactive interface designed for the terminal 101.

In some embodiments, the intelligent head cover 200 further comprises an advising unit 295 and a display unit 290. The advising unit 295 is configured to generate advice information based on sensory data, the advice information including at least one of a hairstyle advice, a dyeing advice, a massage advice, and a health condition advice; or, the advising unit 295 may be further configured to receive the advice information from the terminal. The display unit 290 may be located in the front of the intelligent head cover 200, and the display unit is configured to display the advice information generated by the advising unit 295 or received by the advising unit 295 to the user. In some examples, the display unit 290 includes a touch screen, and the user may select an option in the advice information through the touch screen. For example, the user may select a recommended hairstyle through the touch screen.

In some examples, when the hairstyle advice information is acquired, the intelligent head cover may further execute operations as follows: when the intelligent head cover 200 does not comprise the advising unit 295, the controller 230 generates a default hair cutting instruction based on the sensory data, and the hair cutting instruction is used for controlling the turning ON or OFF of the hair cutting component (for example, the control instruction may be an ON or OFF instruction of a razor and a comb with a corresponding identifier and address); when the intelligent head cover 200 comprises the advising unit 295, the instruction generator 250 comprised in the intelligent head cover may generate the hair cutting instruction according to the sensory data and the hairstyle advice information, and the hair cutting instruction is used for controlling turning ON or OFF of the hair cutting component (for example, the control instruction may be an ON or OFF instruction of a razor and a comb with a corresponding identifier and address).

In some embodiments, the display unit 290 may be an LCD display, and may also be an LED display. In addition, the intelligent head cover 200 may also show the user a progress of hair cutting or dyeing in real time by means of the display unit 290. For example, the display unit 290 may also additionally have an audiovisual-material playback function, to help a person to have a better time during hair cutting by playing an audiovisual material.

Hereinafter, a technical solution according to an embodiment will be further described in conjunction with the intelligent head cover provided in FIG. 3. The plurality of sensors according to this embodiment are a plurality of cameras. FIG. 3 may schematically show positional relation for deploying related devices inside the intelligent head cover; and the related devices may include a hair cutting component 302, a massage unit 303, a display 304, and the like.

As shown in FIG. 3, an intelligent head cover 300 comprises: two cameras 301, a hair cutting component 302, a massage unit 303, a display 304, a communication unit 305, a heating unit 306 and a dyeing component 307. For example, one of the two cameras 301 is located in the front of the interior of the intelligent head cover, and the other is located in the back or on the top of the interior of the intelligent head cover (which is specifically in an upper rear position with reference to FIG. 3). The camera located in the front may simultaneously acquire images that represent information such as a facial form, a hair density and color in the front of the wearer, and the camera located in the upper rear position may acquire images for representing parameter information such as a hair length, color and density in the upper rear position. The hair cutting component 302 includes razors and combs, and the hair cutting component is non-uniformly provided obliquely above the wearer's head and in a lower rear position of the head. The massage unit 3 03 is located in the upper rear position inside the intelligent head cover, and may be turned on or turned off according to a control instruction (for example, a massage instruction). The display 304 is provided in front of the eyes inside the head cover, and the display 304 may display a current hair cutting progress to the wearer in real time, so that the wearer immediately knows whether there is a large deviation in a hair cutting situation. In addition, the display 304 may also play a certain audio-visual program, to help the wearer to spend time for hair cutting more comfortably. In this case, the intelligent head cover 300 needs to have a storage space for storing and playing an audiovisual file, or to have a network interface that receives an audiovisual material in real time through a network. The communication unit 305 is located above the intelligent head cover; and the communication unit 305 may send or receive a signal with a built-in antenna. The heating unit 306 may be provided in a lower rear position of the intelligent head cover; and certainly, it may also be provided in another position where hairs are denser, so as to achieve a better drying effect.

The heating unit 306 shown in FIG. 3 is configured to implement heat generation and preservation in the interior of the head cover. For example, if it is desired to dry the hair, the heating unit 306 is selected to heat up quickly; if it is desired to help the dye to work better on coloring, the heating unit 306 may be set to maintain a certain temperature. For example, the massage unit 303 may perform massage on the head of the wearer according to a preset acupuncture point, wherein a force and time of massage may be controlled by a massage instruction sent by the terminal 101 shown in FIG. 1.

The intelligent head cover shown in FIG. 3 may comprise components or units as follows. A transparent display is provided in the front of the interior of the intelligent head cover. Cameras are provided in a plurality of positions inside the intelligent head cover. Multiple sets of miniature razors and combs are provided in the upper, left and right positions inside the intelligent head cover, a circuit controls the miniature razors or combs whether to operate or not, and each set of razors or combs may move within a certain small range. A plurality of massage units are provided inside the intelligent head cover, which implement massage on the head by vibrations driven by a motor. The intelligent head cover is further provided therein with a plurality of pipes and storage capsules for storing the dye required for dyeing. In addition, the intelligent head cover is provided therein with a plurality of heating units, to implement heating and temperature-preservation functions. In addition, the intelligent head cover 300 may further be connected with an external smart mobile device such as a mobile phone, a wristband, a smart watch, etc., to feed back various types of information to the mobile device. In addition, the intelligent head cover may further be connected with the network through the external smart mobile device, so that data may be updated in real time (for example, latest popular hairstyles may be stored in the intelligent head cover, and then the hairstyles may be sent by the intelligent head cover to the terminal shown in FIG. 1, so as to facilitate the terminal to select a hairstyle suitable for a wearer of the head cover from these hairstyles).

Corresponding to the above-described intelligent head cover, an embodiment of the present disclosure further provides a control method 400A specific to the intelligent head cover 200.

For example, as shown in FIG. 4A, the control method 400A may comprise: step 410, acquiring sensory data related to a user; and step 420, controlling a hairstyle processing component to perform a hairstyle processing action based on a control instruction.

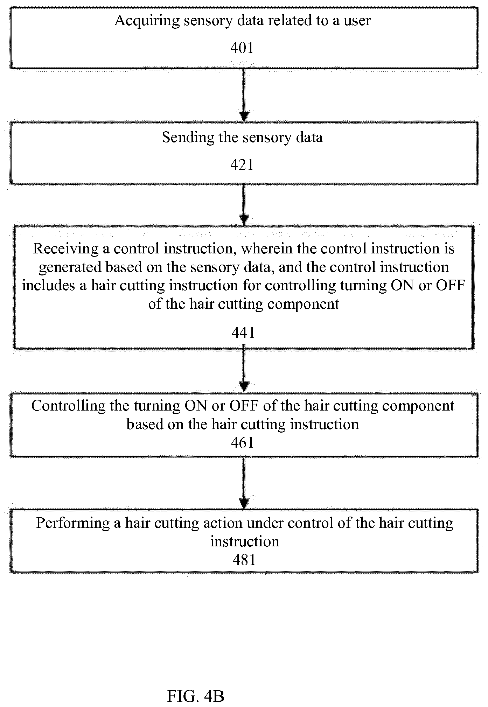

As shown in FIG. 4B, the diagram shows a controlling and processing procedure of the above-described control method 400 specific to the hair cutting component. For example, at least one embodiment of the present disclosure provides a control method 400 of an intelligent head cover, and the control method 400 may comprise: step 401, acquiring sensory data related to a user; step 421, sending the sensory data; step 441: receiving a control instruction or generating the control instruction with an instruction generator of its own, wherein the control instruction is generated based on the sensory data, and the control instruction includes a hair cutting instruction for controlling the turning ON or OFF of the hair cutting component; step 461, controlling the turning ON or OFF of the hair cutting component based on the hair cutting instruction; and step 481: performing a hair cutting action under control of the hair cutting instruction.

The sensory data acquired in the above-described step 401 may include image data acquired with a camera, and may also include temperature data obtained with a temperature sensor, and the like.

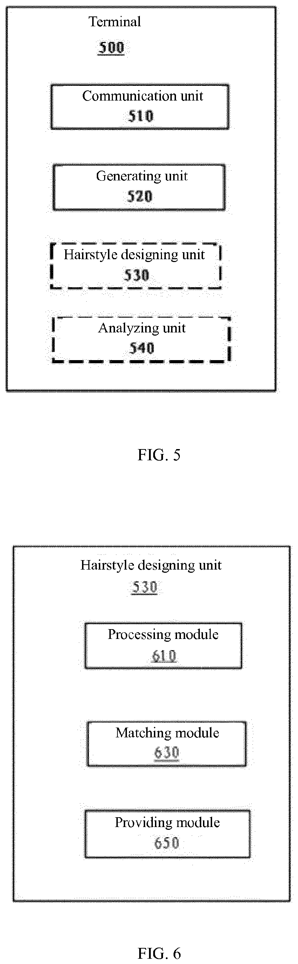

In the above-described step 421, the sensory data is sent to a corresponding device (for example, the terminal shown in FIG. 5), and then the device extracts a facial form, a head form, and other parameter information based on the sensory data, and generates a recommended hairstyle or a control instruction, and the like, based on the extracted related information.

The above-described step 441 is used for receiving a control instruction from a corresponding device (i.e., the terminal shown in FIG. 5). For example, the control instruction may be a hair cutting instruction, a dyeing instruction, a massage instruction, or a heating instruction, and the like.

An embodiment of the present disclosure provides a terminal 500. The terminal 500 may serve as the terminal shown in FIG. 1. The terminal 500 may comprise: a communication unit 510 and a generating unit 520; wherein the generating unit 520 is configured to generate a control instruction according to sensory data, and the control instruction is used for controlling a hairstyle processing component in an intelligent head cover to perform a hairstyle processing action. The communication unit 510 is configured to send the control instruction to the intelligent head cover or receive the sensory data from the intelligent head cover.

In some embodiments, the terminal 500 may further comprise a hairstyle designing unit 530. The hairstyle designing unit 530 is configured to provide a hairstyle advice based on sensory data related to a user.

As shown in FIG. 6, in some embodiments, the hairstyle designing unit 530 may further include: a processing module 610, configured to process sensory data to generate a head form parameter, a facial form parameter or a hair parameter of a user; a matching module 630, configured to match the head form parameter, the facial form parameter or the hair parameter of the user with data of a hairstyle database, to obtain a hairstyle advice including at least one recommended hairstyle; and a providing module 650, configured to provide the user with the hairstyle advice.

In some embodiments, a control instruction at least includes a massage instruction for controlling a massage unit of an intelligent head cover and a heating instruction for controlling a heating unit of the intelligent head cover. Correspondingly, in this case, the generating unit 520 is further configured to generate the hair cutting instruction, the massage instruction and the heating instruction.

In some embodiments, the terminal 500 further comprises an analyzing unit 540. The analyzing unit 540 is configured to: acquire a hair quality parameter of a user according to sensory data, the hair quality parameter including hair color and hair elasticity; and determine the user's health condition according to the hair quality parameter, and provide a health condition advice.

As shown in FIG. 7A, a schematic diagram of a user interactive interface used in the terminal 500 of FIG. 5 is provided, different services such as hairstyle design, head cover management, health detection, and massage may be provided to the user through the interface schematic diagram, and the user may also implement interaction with an application through the interactive interface of FIG. 7A, to further select a certain function or service provided by the terminal 500 or the intelligent head cover.

For example, when the user clicks on a hairstyle design function shown in FIG. 7A, the terminal 500 may further provide a display interface as shown in FIG. 7B, and the terminal may provide an effect picture of a recommended hairstyle to the user though the display interface of FIG. 7B. In addition, the display interface may also show a color related to the recommended hairstyle to the user, and may accept information input by the user at a same time. The input information may allow the user to select a certain color from the recommended colors. In this way, it is possible to allow the user to actively select elements related to the designed hairstyle, so as to better meet the user's needs for differentiation.

For example, when the user clicks on a hair quality analysis option in the interface of FIG. 7A, the terminal 500 provides a display interface shown in FIG. 7C, and through the information displayed on the interface, it can be known that: hair quality analysis performed on the user may include hair quality evaluation and hair quality diagnosis on the user's hair. For example, health analysis may be further performed on the user by hair quality diagnosis, so as to provide advices on various aspects such as a reasonable healthy diet. For example, the hair quality analysis may be performed in conjunction with parameters as follows: a hair color, a hair density, a hair elasticity, a hair moisture and brightness, and the like. For example, the health condition may be obtained in aspects as follows: metal content included in the hair, hair trace elements, and the like. For example, whether or not the user has zinc deficiency may be derived by judging a relationship between zinc content in the hair and a set threshold value, so as to further prompt the user to supplement zinc.

Hereinafter, a processing method 800 that may be used in the terminal 500 will be described in detail below in conjunction with FIG. 8.

As shown in FIG. 8, the processing method 800 may comprise: step 801, receiving sensory data related to a user from an intelligent head cover; step 811, providing a hairstyle advice according to the sensory data; step 812, generating a control instruction according to the sensory data, wherein the control instruction includes a hair cutting instruction for controlling the turning ON or OFF of a hair cutting component in the intelligent head cover; and step 813, sending the control instruction to the intelligent head cover.

In some embodiments, the processing method 800 may further comprise: acquiring a user's hair quality parameter according to the sensory data, the hair quality parameter including a hair color and hair elasticity; and acquiring the user's health condition according to the hair quality parameter, and providing an advice for improvement.

For example, the sensory data in step 801 includes: image data collected by a camera, temperature data collected by a temperature sensor, and the like. For example, the image data collected by the camera inside the intelligent head cover may be received through a wireless network.

For example, in step 811, the sensory data acquired in step 801 is analyzed firstly, and then a head form, a facial form and a hair density of the user, and other parameter information are further obtained according to an analysis result. Then a hairstyle advice may be provided according to the parameter information. Parameters related to hair cutting may further include current hair length information, hair color information or skin color information, and the like, of a wearer of the head cover. For example, when a plurality of cameras acquires images of the wearer of the head cover, parameter information of hair cutting below may be obtained by analyzing the images: fair complexion, relatively thin hair, relatively long hair, and a round face. A recommended hairstyle may be generated in combination with these parameters, and then the generated hairstyle advice is displayed to the wearer of the head cover by text or picture: a pageboy style, dyed dark red.

For example, in step 812, the control instruction may be generated based on the sensory data acquired from step 801. The sensory data may further include feedback information of a hair cutting situation in a hair cutting process, and the control instruction generated in this case may include a hair cutting instruction for controlling the turning on or off of one or more certain razors, may also include a dyeing instruction for controlling a storing unit and a pipe to perform dyeing, and may also include a heating instruction for controlling a heating unit to generate heat timely. The control instruction may further include a massage instruction for controlling a massage unit.

For example, in step 813, the generated control instruction may be sent by a wireless network. A control instruction that requires high security and timeliness may be sent in a reliable instruction transmission approach, to avoid damage to the user. With respect to a control instruction that does not require high security and timeliness, it only needs to be sent out. For example, reliable transmission of the instruction that requires high security may be ensured by setting feedback confirmation information (for example, controlling a certain razor being turned on or controlling the heating unit heating).

For example, in step 811, the parameter information obtained after analyzing the sensory data may further include: hair quality parameter information. In this case, the method 800 may further comprise: generating health status indication information according to the hair quality parameter information. Finally, the status indication information is sent to a prompting unit (e.g., a display) and displayed.

In summary, the embodiments of the present disclosure may implement: acquiring the images based on a camera group detection system inside the intelligent head cover; and then determining the hair length, distribution, and other appearances of respective head regions of the person wearing the head cover according to these images, and feeding the same back to the display screen right in front of the intelligent head cover or the external mobile device. Reasonable matching is performed through the external mobile device according to various aspects of factors, such as the head form, the facial form and the hair condition, in combination with the database having various hairstyles stored therein, so as to propose various suitable hairstyle advices; after selecting an appropriate hairstyle, confirmation can be performed on the mobile device, so that the intelligent head cover automatically cuts out the selected hairstyle. Further, the intelligent head cover may also provide the user with the massage function and even functions such as perming and dyeing. The intelligent head cover detects and analyzes the hair quality condition, feeds back the user's hair and health condition, and further provides a reasonable advice for improvement. In addition, the present disclosure is not only applicable to a liquid crystal display panel, but also applicable to E-paper, an OLED, and other display products.

That is to say, the present disclosure provides an intelligent head cover based on the Internet of Things; the intelligent head cover comprises the display screen located in the front, cameras distributed in a plurality of positions of the intelligent head cover, miniature razors, combs and massagers distributed in a mesh structure, and the like. In addition, the intelligent head cover further comprises pipes and storage capsules distributed in a mesh structure, as well as a miniature heating unit. The intelligent head cover (for example, which may be the intelligent head cover shown in FIG. 2) is interconnected with the mobile device (for example, the device shown in FIG. 4) with the Internet of Things, detects the head form, the facial form, and the like of the user with the cameras, and selects the appropriate hairstyle by matching. The intelligent head cover may further cut out the selected hairstyle, and may further implement analysis and detection of the hair quality, to learn the heath condition of the user.

Various embodiments described herein may be implemented in a computer-readable medium with, for example, computer software, hardware, or any combination thereof. With respect to implementation with hardware, the embodiments described herein may be implemented with at least one of an application specific integrated circuit (ASIC), a digital signal processor (DSP), a digital signal processing device (DSPD), a programmable logic device (PLD), a field programmable gate array (FPGA), a processor, a controller, a microcontroller, a microprocessor, and an electronic unit designed to execute the functions described herein; in some cases, such embodiments may be implemented in a processor unit 130. With respect to implementation by software, embodiments such as procedures or functions may be implemented with a separate software module allowing execution of at least one function or operation. Software codes may be implemented by a software application program (or a program) written in any suitable programming language, and the software codes may be stored in a memory 140 and executed by the processor unit 130.

A person skilled in the art may be aware that the various exemplary units and algorithm steps described in conjunction with the embodiments disclosed herein can be implemented by electronic hardware, or a combination of computer software and electronic hardware. Whether the functions are executed by hardware or software depends on particular applications and design constraint conditions of the technical solutions. A person skilled in the art can use different methods to implement the described functions for every particular application, but it should not be considered that the implementation goes beyond the scope of the present disclosure.

Those skilled in the art are able to clearly understand that, for easy and concise description, the specific working processes of the apparatus and the unit described previously may refer to the corresponding processes in the method embodiment, and are not described herein.

In the embodiments provided in the present disclosure, it should be understood that the disclosed device and method may be implemented in other modes. For example, the device embodiment described above is merely exemplary. For example, the division of units is merely a division of logical functions and there may be other division modes in actual applications. For example, multiple units or components may be combined or may be integrated to another device, or some characteristics may be ignored or not executed.

The units described as separate parts may or may not be physically separate, and parts displayed as units may or may not be physical units. A part or all of the units may be selected according to the actual needs to achieve the objectives of the solutions of the embodiments.

When being implemented in the form of a software function unit and sold or used as a stand-alone product, the functions may be stored in a computer-readable storage medium. Based on such understanding, the essence of the technical solutions of the present disclosure, or part that makes contributions to the prior art, or part of the technical solution may be embodied in the form of a software product. The computer software product may be stored in a storage medium, and incorporates several instructions for instructing a computer device (for example, personal computer, server, or network device) to execute all or part of the steps of the method in any embodiment of the present disclosure. The storage medium may be any medium that is capable of storing program codes, such as a USB flash drive, a removable hard disk, a Read-Only Memory (ROM), a Random Access Memory (RAM,), a magnetic disk, or an optical disk.

What are described above is related to the illustrative embodiments of the disclosure only and not limitative to the scope of the disclosure; any changes or replacements easily for those technical personnel who are familiar with this technology in the field to envisage in the scopes of the disclosure, should be in the scope of protection of the present disclosure. Therefore, the scopes of the disclosure are defined by the accompanying claims.

The present application claims the priority of the Chinese Patent Application No. 201710001153.1 filed on January 3, 2017, which is incorporated herein by reference in its entirety as part of the disclosure of the present application.

## Claims

1. An intelligent head cover, comprising:
a sensor, configured to acquire sensory data related to a user.
a hairstyle processing component; and
a controller, configured to control the hairstyle processing component to perform a hairstyle processing action based on a control instruction, wherein:
the control instruction is generated based on the sensory data.

2. The intelligent head cover according to claim 1, further comprising a communication unit, wherein:
the communication unit is configured to send the sensory data and receive the control instruction, and the control instruction includes a hair cutting instruction for controlling turning on or off of the hairstyle processing component.

3. The intelligent head cover according to claim 1, further comprising an instruction generator, wherein:
the instruction generator is configured to generate the control instruction based on the sensory data, the control instruction includes a hair cutting instruction for controlling turning on or off of the hairstyle processing component.

4. The intelligent head cover according to claim 1, further comprising a communication unit, an instruction generator and a selector, wherein:
the communication unit is configured to establish connection with a terminal;
the selector is configured to:
instruct the communication unit to receive the control instruction from the terminal, when the communication unit successfully establishes connection with the terminal;
instruct the instruction generator to generate the control instruction, when the communication unit fails to establish connection with the terminal;
when the communication unit successfully establishes connection with the terminal, the communication unit is further configured to send the sensory data to the terminal and receive the control instruction from the terminal, the control instruction is generated based on the sensory data by the terminal; or
when the communication unit fails to establish connection with the terminal, the instruction generator is configured to generate the control instruction based on the sensory data; and
the control instruction includes a hair cutting instruction for controlling turning on or off of the hairstyle processing component.

5. The intelligent head cover according to any one of claims 1 to 4, wherein:
the plurality of sensors includes: a plurality of cameras;
the sensory data includes: images of the user's head taken by the plurality of cameras; and
the images of the user's head include a combination of one or more images of a head form, a facial form and hair distribution of the user.

6. The intelligent head cover according to claim 5, wherein:
at least one of the plurality of cameras is located in the front of an interior of the head cover, the front is a position close to a face when the intelligent head cover is worn; and
at least one of the plurality of cameras is located in the back or on the top of the interior of the intelligent head cover, the back is a position away from the face when the intelligent head cover is worn, and the top is a position close to a head vertex when the intelligent head cover is worn.

7. The intelligent head cover according to any one of claims 1 to 6, wherein:
the plurality of sensors includes a temperature sensor; and
the sensory data further includes a temperature inside the intelligent head cover sensed by the temperature sensor.

8. The intelligent head cover according to any one of claims 2 to 4, wherein the hairstyle processing component includes a hair cutting component;
the hair cutting component includes: razors or combs distributed on an inner surface of the intelligent head cover;
the razors are configured to: cut the user's hair based on the hair cutting instruction; and
the combs are configured to: comb the user's hair based on the hair cutting instruction.

9. The intelligent head cover according to claim 8, wherein the hair cutting instruction further includes position information of the razors and the combs.

10. The intelligent head cover according to any one of claims 1 to 9, wherein the hairstyle processing component includes a dyeing component;
the dyeing component further includes: pipes and a storing unit distributed in a mesh structure on an inner surface of the intelligent head cover;
the storing unit is configured to store a dyeing material.

11. The intelligent head cover according to claim 10, wherein:
the control instruction further includes a dyeing instruction;
the controller is configured to control opening or closure of the storing unit based on the dyeing instruction; and
the storing unit is further configured to provide the dyeing material to the pipes when opened.

12. The intelligent head cover according to any one of claims 1 to 11, wherein a heating unit is configured to generate heat and provide temperature preservation inside the intelligent head cover under control of a heating instruction.

13. The intelligent head cover according to any one of claims 1 to 12, further comprising:
a massage unit located inside the intelligent head cover, and configured to perform massage on the user's head under control of a massage instruction.

14. The intelligent head cover according to any one of claims 1 to 13, further comprising:
a hair quality detecting unit, configured to detect the user's hair quality.

15. The intelligent head cover according to any one of claims 1 to 14, further comprising an advising unit and a display unit, wherein:
the advising unit is configured to generate advice information based on the sensory data, the advice information including at least one of a hairstyle advice, a dyeing advice, a massage advice, and a health condition advice; or, the advising unit is configured to receive the advice information; and
the display unit is located in the front of the intelligent head cover, and is configured to display the advice information to the user.

16. A control method of the intelligent head cover according to any one of claims 1 to 15, comprising:
acquiring sensory data related to a user; and
controlling a hairstyle processing component to perform a hairstyle processing action based on a control instruction.

17. A terminal, comprising:
a communication unit, configured to receive sensory data related to a user; and
a generating unit, configured to generate a control instruction according to the sensory data, the control instruction being used for controlling a hairstyle processing component in an intelligent head cover to perform a hairstyle processing action; wherein:
the communication unit is further configured to send the control instruction to the intelligent head cover.

18. The terminal according to claim 17, further comprising a hairstyle designing unit, configured to provide a hairstyle advice based on the sensory data related to the user,
wherein the hairstyle designing unit includes:
a processing module, configured to process the sensory data to generate a head form parameter, a facial form parameter or a hair parameter of the user; and
a matching module, configured to match the head form parameter, the facial form parameter or the hair parameter of the user with data of a hairstyle database, to obtain a hairstyle advice including at least one recommended hairstyle; and
a providing module, configured to provide the user with the hairstyle advice.

19. The terminal according to any of claims 17 to 18, wherein:
the control instruction includes a massage instruction for controlling a massage unit of the intelligent head cover and a heating instruction for controlling a heating unit of the intelligent head cover; and
the generating unit is further configured to generate the hair cutting instruction, the massage instruction and the heating instruction.

20. The terminal according to any one of claims 17 to 19, further comprising an analyzing unit, configured to:
acquire a hair quality parameter of the user according to the sensory data, the hair quality parameter including a hair color and a hair elasticity; and
acquire the user's health condition according to the hair quality parameter, and provide a health condition advice.
